Europäisches Patentamt

European Patent Office

Office européen des brevets

(19)

(11) Publication number: **0 139 047**
A1

(12)

# EUROPEAN PATENT APPLICATION

(21) Application number: 83201496.3

(22) Date of filing: 18.10.83

(51) Int. Cl.⁴: **C 07 D 499/00**

(43) Date of publication of application:
02.05.85 Bulletin 85/18

(84) Designated Contracting States:
NL

(71) Applicant: Gist - Brocades N.V.
Wateringseweg 1 P.O. Box 1
NL-2600 MA Delft(NL)

(72) Inventor: Kapur, Jagdish Chander
Roland Holstlaan 799
NL-2624 KB Delft(NL)

(72) Inventor: Fasel, Herman Pieter
Van der Haertstraat 19
NL-2613 XZ Delft(NL)

(74) Representative: Schmieman, Johannes Hendrik et al,
Gist-Brocades N.V. Patents & Trademarks Department
Wateringseweg 1 P.O. Box 1
NL-2600 MA Delft(NL)

(54) Process for the preparation of 6,6-dibromopenicillanic acid 1,1-dioxide.

(57) The invention relates to a process for the preparation of 6,6-dibromopenicillanic acid 1,1-dioxide by reaction of 6-bêta-aminopenicillanic acid 1,1-dioxide with a nitrosating agent in the presence of an inorganic or strong organic acid and bromine. The invention is characterized in that the reaction is performed in the presence of an alcohol.

EP 0 139 047 A1

0139047

EUR-2379

Process for the preparation of 6,6-dibromopenicillanic
acid 1,1-dioxide

The invention relates to a process for the preparation of 6,6-dibromopenicillanic acid 1,1-dioxide by reaction of 6-bêta-aminopenicillanic acid 1,1-dioxide with a nitrosating agent in the presence of an inorganic or strong organic acid and bromine.

The presumed association between the resistance of certain bacteria to bêta-lactam antibiotics has led to an intensive search for bêta-lactamase inhibitors.

It is known from Dutch patent application 7806126 that penicillanic acid 1,1-dioxide and salts and esters thereof, have useful pharmacological properties, for example as effective inhibitors of several types of bêta-lactamases present in various kinds of bacteria. In the beforementioned Dutch application a process is described for the preparation of penicillanic acid 1,1-dioxide and salts and esters thereof by oxidation of penicillanic acid.

Another process for the preparation of penicillanic acid 1,1-dioxide is described in Dutch patent application 8001285. In this application penicillanic acid 1,1-dioxide is prepared by diazotisation-bromination of 6-aminopenicillanic acid followed by oxidation of the formed 6,6-dibromopenicillanic acid into 6,6-dibromo-penicillanic acid 1,1-dioxide and dehalogenation of the latter compound.

In European patent application 83200541 a process is described for the preparation of a mixture of 6,6-dibromopenicillanic acid 1,1-dioxide and 6-alpha-bromo penicillanic acid 1,1-dioxide by diazotisation-bromination of 6-aminopenicillanic acid 1,1-dioxide. The relative amount of dibromo compound in the mixture usually varies from 80-90%, the relative amount of monobromo compound from 10-20%. The mixture of the bromocompounds thus prepared can be reduced into penicillanic acid 1,1-dioxide for instance in the way as described in European patent application 83200542.

In the above mentioned European patent application 83200541 the mixture of 6,6-dibromopenicillanic acid 1,1-dioxide and 6-alpha-bromopenicillanic acid 1,1-dioxide is prepared by reaction of 6-aminopenicillanic acid 1,1-dioxide with a nitrosating agent in the presence of hydrogen bromide and bromine. The reaction is performed at a temperature between $-20°C$ and $30°C$ with at least an equimolar amount of a nitrosating agent in the presence of 1 to 5 equivalents of a strong inorganic or organic acid in solution or suspension of a mixture of water and a partly or completely water-miscible organic solvent medium, the amount of water present being from 1 to 20% by volume, containing hydrogen bromide and bromine in at least equimolar amounts. Optionally an auxiliary agent which facilitates the bromination of the diazotized intermediate in an amount varying from 10% to at least an equimolar amount of the aminopenicillanic acid 1,1-dioxide starting material can be used. The maximum yield of 6,6-dibromopenicillanic acid 1,1-dioxide and 6-alpha-bromopenicillanic acid in the examples of the above mentioned European patent application calculated on the starting 6-amino-penicillanic acid 1,1-dioxide did not exceed the 70%, in spite of the extensive disclosure.

- 3 -                                          0139047

It has now been found that pure 6,6-dibromo-
penicillanic acid can be prepared in a considerably higher
yield than described in the abovementioned European patent
application 83200541 by the addition of a small amount of
an alcohol to the reaction mixture of the diazotisation-
bromination reaction of 6-aminopenicillanic acid 1,1-
dioxide. The present invention, therefore, relates to a
process for the preparation of 6,6-dibromopenicillanic
acid 1,1-dioxide by reaction of 6-bêta-aminopenicillanic
acid 1,1-dioxide with a nitrosating agent in the presence
of an inorganic or strong organic acid and bromine,
characterized in that an alcohol is present in the
reaction mixture.

When using the process of the present invention it
is possible to prepare pure 6,6-dibromopenicillanic acid
1,1-dioxide in a yield of 90%. In comparison with the
process described in European patent appliction 83200541 a
relative improvement of the amount of bromo compounds of
about 30% is reached. This means that the cost price of
the bromo compounds as prepared by the older process will
be 30% higher than the cost price of the dibromo compound
as prepared by the process of the present invention. The
improvement of the yield is illustrated for instance by
examples 3 and 6. Keeping all the other reaction
conditions the same, the yield of 6,6-dibromopenicillanic
acid 1,1-dioxide improves from 53 to 90 % by the addition
of a small amount of methanol.

Furthermore the recovery procedure in the present
process is usually more simple than in the process
described in the beforementioned European patent
application 83200541. In that application it is neccesary
to remove the water-miscible solvent by azeotropic
destillation or by evaporation _in vacuo_, as otherwise the
extraction of the product from the aqueous layer would
give problems. As the reaction of the present invention can be performed in methylene chloride it is not neccesary any more to remove the water-miscible solvent, which is an important feature in a large-scale chemical process.

It is another advantage of the present application that 6,6-dibromo penicillanic acid 1,1-dioxide of a high purity is obtained. The ultimately desired product, i.e. penicillanic acid 1,1-dioxide, will therefore also have a high purity, which is an important feature of compounds to be used for the preparation of pharmaceutical compositions. Also the fact that the process of the present invention results in pure 6,6-dibromo penicillanic acid 1,1-dioxide and is not mixed with an amount of 6-alpha-bromo-penicillanic acid 1,1-dioxide has a positive influence on the quality of the end product.

The reaction is carried out by addition of bromine, aqueous hydrobromic acid and the alcohol to a solution or suspension of 6-aminopenicillanic 1,1-dioxide acid in an organic solvent followed by the addition of the nitrosating agent. After stirring for 5 minutes to 1 hour an eventual excess of bromine is removed for instance by addition of an aqueous solution of sodium bisulfite. Extraction with an organic solvent, drying in the usual way and evaporation of the solvent results in the desired 6,6-dibromopenicillanic acid 1,1-dioxide.

Examples of alcohols which can be used in the process of the present invention are methanol, ethanol, propanol, i-propanol, t-butylalcohol, pentanol, hexanol, cyclohexanol, benzylalcohol, 1,2-propanediol and glycerol. However, the scope of the present is not limited to the above mentioned alcohols, but can also be extended to other alcohols. Preferably methanol is used. The amount of alcohol may vary between 1 and 10 equivalents calculated on the starting 6-aminopenicillanic acid 1,1-dioxide. Preferably 2-6 equivalents of the alcohol are used, more preferably 4 equivalents. Examples of inorganic and strong organic acids are hydrobromic acid, hydrochloric acid, sulphuric acid, phosphoric acid, aminosulphonic acid, chloroacetic acid, dichloroacetic and trifluoroacetic acid. Preferably hydrobromic acid is used.

Suitable organic solvents in which the reaction can be carried out are inert organic solvents, for example methylene chloride, chloroform and acetonitril can be used. Preferably methylene chloride is used.

The reaction can be carried out at a temperature between -20° and 30°C, preferably between -10 and 15°C.

The nitrosating agent to be used in the reaction can be an alkali metal nitrite or an alkylnitrite. Preferably sodium nitrite is used.

The starting 6-aminopenicillanic acid 1,1-dioxide can be prepared as described in European Patent 0030771.

The following non-limitative examples illustrate the present invention.

General remarks:

1. Alcohol-free dichloromethane has been used in all the examples.

2. The purity of 6,6-dibromopenicillanic acid 1,1-dioxide has been determined through 60 MHz-NMR spectra using either maleic acid as the reference in dimethylsulfoxide-$d_6$ or 2,6-dichloroacetophenone as the reference in acetone-$d_6$.

- 6 -

0139047

## Example 1

To a suspension of 6-bêta-aminopenicillanic acid 1,1-dioxide (6.2 g; purity by HPLC 90%; 22.5 mmol) in dichloromethane (75 ml) cooled to about -5° to -0°C was added a solution of bromine (6.0 g; 37.5 mmol) in dichloromethane (25 ml), hydrobromic acid (7.1 ml; 64.0 mmol) and methanol (2 ml; 49.4 mmol). Sodium nitrite (2.05 g; 29.7 mmol) was added portionwise over a period of 10-15 min. During the addition of sodium nitrite the reaction mixture was maintained at 0° to 5°C. The contents were further stirred for 30 min. at the same temperature. A solution of sodium bisulfite (35 ml; 10% aqueous) was added dropwise at 0° to 5° till the bromine colour was discharged. The reaction mixture was extracted with chloroform (4 x 200 ml). The combined extracts were washed with brine (2 x 100 ml) and dried over anhydrous magnesium sulfate. Removal of the solvent, washings with n-hexane and drying under reduced pressure resulted in 7.29 g of 6,6-dibromopenicillanic acid 1,1-dioxide.
IR (KBr): 2700-3300, 1811, 1740, 1333 $cm^{-1}$ ; NMR (DMSO-$d_6$, delta-values in ppm, TMS, 60 MHz): 1.43 (s, 3H, $CH_3$), 1.53 (s, 3H, $CH_3$), 4.72 (s, 1H, $C^3H$), 6.02 (s, 1H, $C^5H$). Purity 99.1%, thus giving a yield of 82.2 %. The purity was determined through 60 MHz NMR spectroscopy using maleic acid as the reference.

## Example 2

The reaction was carried out as described in example 1, however, the amount of methanol used was 3 ml (74.1 mmol) in stead of 2 ml. Isolated yield of 6,6-dibromopenicillanic acid 1,1-dioxide = 7.93 g (purity 98.5%), thus giving a yield of 88.9 %.

## Example 3

The reaction was carried out as described in example 1, however, the amount of methanol used was 4 ml (98.75 mmol) in stead of 2 ml. Isolated yield of 6,6-dibromopenicillanic acid 1,1-dioxide = 7.98 g (purity 99.1%), thus giving a yield of 89.9%.

## Example 4

The reaction was carried out as described in example 1, however, the amount of methanol used was 5 ml (123.4 mmol) in stead of 2 ml. Isolated yield of 6,6-dibromopenicillanic acid 1,1-dioxide = 8.09 g (purity 90.9 %), thus giving a yield of 83.6%.

## Example 5

The reaction was carried out as described in example 1, however, the amount of methanol used was 6 ml (148.1 mmol) in stead of 2 ml. Isolated yield of 6,6-dibromopenicillanic acid 1,1-dioxide = 8.23 g (purity 92.6%), thus giving a yield of 86.7%.

## Example 6

The reaction was performed as described in example 1, however, the amount of hydrobromic acid was 7.46 ml (67 mmol) and no methanol was used. Isolated yield of 6,6-dibromopenicillanic acid 1,1-dioxide = 4.72 g (purity 98.8%), thus giving a yield of 53.1%.

## Example 7

The reaction was carried out as described in example 6, however, the amount of methanol used was 0.1 ml (2.47 mmol). Isolated yield of 6,6-dibromopenicillanic acid 1,1-dioxide = 5.1 g (purity 97.15%), thus giving a yield of 57.2%.

## Example 8

The reaction was carried out as described in example 6, however, the amount of methanol used was 0.4 ml (9.9 mmol). Isolated yield of 6,6-dibromopenicillanic acid 1,1-dioxide = 5.76 g (purity 94.85%), thus giving a yield of 62.3%

## Example 9

The reaction was carried out as described in example 6, however, the amount of methanol used was 1 ml (24.7 mmol). Isolated yield of 6,6-dibromopenicillanic acid 1,1-dioxide = 7.08 g (purity 99.4%), thus giving a yield of 80.1%.

## Example 10

The reaction was carried out as described in example 6, however, the amount of methanol used was 2 ml (49.4 mmol). Isolated yield of 6,6-dibromopenicillanic acid 1,1-dioxide = 7.72 g (purity 96.7%), thus giving a yield of 85.2%.

## Example 11

The reaction was carried out as described in example 1, however, the amount of methanol used was 3 ml (74.06 mmol). Isolated yield of 6,6-dibromopenicillanic acid 1,1-dioxide = 7.54 g (purity 98.6%), thus giving a yield of 84.6%.

## Example 12

The reaction was carried out as described in example 6, however, the amount of methanol used was 4 ml (98.8 mmol). Isolated yield of 6,6-dibromopenicillanic acid 1,1-dioxide = 8.00 g (purity 96.97%), thus giving a yield of 88.3%.

## Example 13

The reaction was carried out as described in example 6, however, the amount of methanol used was 5 ml (123.4 mmol). Isolated yield of 6,6-dibromopenicillanic acid 1,1-dioxide = 8.17 g (purity 95.7%), thus giving a yield of 89%.

## Example 14

The reaction was carried out as described in example 6, however, the amount of methanol used was 6 ml (148.1 mmol). Isolated yield of 6,6-dibromopenicillanic acid 1,1-dioxide = 8.01 g (purity 97.7%), thus giving a yield of 89%.

## Example 15

The reaction was carried out as described in example 1, however, the amount of methanol used = 7 ml (172.8 mmol).

Isolated yield of 6,6-dibromopenicillanic acid 1,1-dioxide = 7.10 g (purity 94.3%), thus giving a yield of 77%. The mother liqor from the isolated product was concentrated in vacuum and dried. NMR analyses of the residue showed that it contained 0,4 g of 6,6-dibromopenicillanic acid 1,1-dioxide = 5.3%, thus giving a total yield of 82.3%.

## Example 16

The reaction was carried out as described in example 6, however, methanol (75 ml) was used as solvent instead of methylene chloride. Bromine (5.99 g, 37.5 mmol) was added as solution in methanol (5 ml). After neutralization of excess bromine, the extractions with chloroform were carried out at pH 3.5. Isolated yield of the crude product = 5.83 g. The NMR spectrum of the crude product showed the presence of 6,6-dibromopenicillanic acid 1,1-dioxide, 6-alpha-bromopenicillanic acid 1,1-dioxide and 6-bromo-6-methoxypenicillanic acid 1,1-dioxide (molar ratio: 2.8: 6.9: 9.5 respectively) and cleaved product(s). The amount of these compounds was determined through 60 MHz NMR spectroscopy using maleic acid as the reference (yield = 13.05%; 26.95% and 41.05% respectively), thus giving a yield of 6,6-dibromopenicillanic acid 1,1-dioxide = 8.7%; 6-alpha-bromopenicillanic acid 1,1-dioxide = 22.4% and 6-bromo-6-methoxypenicillanic acid 1,1-dioxide = 31.1%. The structure of 6-bromo-6-alpha-methoxypenicillanic acid 1,1-dioxide was confirmed by identification of the corresponding methyl ester as described below:

3 g of the above crude bromo-acids were taken in ether and cooled to 0°C. Then diazomethane (solution in ether) was added till the yellow colour persisted. The mixture was stirred at the same temperature for 45 min. The excess of diazomethane was destroyed by addition of glacial acetic acid. The solvent was removed under reduced pressure. The resulting product was taken up in ethyl acetate (60 ml), water (10 ml) was added and the solution was cooled to about 0°C. Then with 1N NaOH, the pH was brought to 7.0. The organic layer was separated, washed with brine, dried over anhydrous magnesium sulfate and the solvent was removed under reduced pressure to a thick oily liquid (2.59 g) containing a mixture of the methyl esters of 6,6-dibromopenicillanic acid 1,1-dioxide, 6-alpha-bromo-penicillanic acid 1,1-dioxide and 6-bromo-6-alpha-methoxy-penicillanic acid 1,1-dioxide as the main products.

2.2 g of this mixture was chromatographed by means of HPLC (Merck Column C) using ethylacetate-$\underline{n}$-hexane (1:3) as the eluent. The fractions containing the 6-bromo-6-alpha-methoxypenicillanic acid 1,1-dioxide methyl ester were collected and evaporated under reduced pressure to a solid product, yield = 0.889 g. IR (CHCl$_3$) 1810, 1760, 1339 cm$^{-1}$. NMR (CDCl$_3$): delta-values in ppm. TMS, 60 MHz) 1.42 (s, 3H), 1.63 (s, 3H), 3.68 (s, 3h), 3.88 (s, 3H), 4.53 (s, 1h), 4.30 (s, 1H). Mass spectrum m/e 355.357.

## Example 17

The reaction was carried out as described in example 1, however, the amount of HBr used was 7.83 ml (70.4 mmol). Isolated yield of 6,6-dibromopenicillanic acid 1,1-dioxide = 7.76 g (purity 98.1%), thus giving a yield of 86.6%.

### Example 18

The reaction was carried out as described in example 17, however, the amount of methanol used was 3 ml (74.1 mmol). Isolated yield of 6,6-dibromopenicillanic acid 1,1-dioxide = 7.73 g (purity 93.75%), thus giving a yield of 82.5%.

### Example 19

The reaction was carried out as described in example 17, however, the amount of methanol used was 4 ml (98.8 mmol). Isolated yield of 6,6-dibromopenicillanic acid 1,1-dioxide = 7.96 g (purity 99.8%), thus giving a yield of 90.3%.

### Example 20

The reaction was carried out as described in example 17, however, the amount of methanol used was 5 ml (123.4 mmol). Isolated yield of 6,6-dibromopenicillanic acid 1,1-dioxide = 8.01 g (purity 96.65%), thus giving a yield of 88.1%.

### Example 21

The reaction was carried out as described in example 17, however, the amount of methanol used was 6 ml (148.13 mmol). Isolated yield of 6,6-dibromopenicillanic acid 1,1-dioxide = 8.09 g (purity 96.9%), thus giving a yield of 89.1%.

Example 22

The reaction was carried out as described in example 1, however, the amount of methanol used was 4 ml (98.8 mmol) and the amount of hydrobromic acid was 1 ml (8.98 mmol). Isolated yield of 6,6-dibromopenicillanic acid 1,1-dioxide = 5.70 g (purity 83.6%), thus giving a yield of 54.8%.

Example 23

The reaction was carried out as described in example 22, however, the amount of hydrobromic acid was 2 ml (17.96 mmol). Isolated yield of 6,6-dibromopenicillanic acid 1,1-dioxide = 7.43 g (purity 87.95%), thus giving a yield of 75.2%.

Example 24

The reaction was carried out as described in example 22, however, the amount of hydrobromic acid was 3 ml (26.94 mmol). Isolated yield of 6,6-dibromopenicillanic acid 1,1-dioxide = 7.63 g (purity 89.55%), thus giving a yield of 78.6%.

Example 25

The reaction was carried out as described in example 22, however, the amount of hydrobromic acid was 4.63 ml (41.56 mmol). Isolated yield of 6,6-dibromopenicillanic acid 1,1-dioxide = 7.81 g (purity 93.95%), thus giving a yield of 84.4%.

## Example 26

The reaction was carried out as described in example 22, however, the amount of hydrobromic acid was 5.3 ml (47.56 mmol). Isolated yield of 6,6-dibromopenicillanic acid 1,1-dioxide = 7.77 g (purity 95.7%), thus giving a yield of 85.6%.

## Example 27

The reaction was carried out as described in example 22, however, the amount of hydrobromic acid was 6.4 ml (57.56 mmol). Isolated yield of 6,6-dibromopenicillanic acid 1,1-dioxide = 7.84 g (purity 94.2%), thus giving a yield of 85%.

## Example 28

The reaction was carried out as described in example 22, however, the amount of hydrobromic acid was 9.17 ml (82.35 mmol). Isolated yield of 6,6-dibromopenicillanic acid 1,1-dioxide = 7.79 g (purity 97.35%), thus giving a yield of 86.1%.

## Example 29

Performed as example 1 using 6-bêta-aminopenicillanic acid 1,1-dioxide (31 g; purity by HPLC = 90%; 112.5 mmol), dichloromethane (375 ml), bromine (30 g; 187.5 mmol) in dichloromethane (125 ml), hydrobromic acid (35.4 ml; 318 mmol), methanol (20 ml; 493.8 mmol), sodium nitrite 810.25 g; 148.5 mmol), sodium metabisulfite (18 g in 180 ml water) and brine (2 x 500 ml).
Isolated yield of 6,6-dibromopenicillanic acid 1,1-dioxide 39.65 g (purity 99.3%), thus giving a yield of 89.6%.

## Example 30

The reaction was carried out as described in example 1, however, the amount of methanol used was 4 ml (98.75 mmol), the amount of hydrobromic acid was 7.46 ml (67 mmol) and pentylnitrite, 3.95 ml (29.7 mmol), was used instead of sodium nitrite. Isolated yield of 6,6-dibromopenicillanic acid 1,1-dioxide = 7.7 g (purity = 93.75%), thus giving a yield of 75.5%.

Example 30

CLAIMS

1. Process for the preparation of 6,6-dibromo-penicillanic acid 1,1-dioxide by reaction of 6-bêta-amino-penicillanic acid 1,1-dioxide with a nitrosating agent in the presence of an inorganic or strong organic acid and bromine, characterized in that an alcohol is present in the reaction mixture.

2. Process according to claim 1, characterized in that the alcohol is methanol.

3. Process according to claim 1-2, characterized in that the amount of methanol is 1 to 10 equivalents, preferably 2-6 equivalents, more preferably 4 equivalents.

4. Process according to claims 1-3, characterized in that the nitrosating agent is an alkali metal nitrite, preferably sodium nitrite.

5. Process according to claim 1-3, characterized in that the nitrosating agent is an alkylnitrite.

6. Process according to claims 1-5, characterized in that the inorganic acid is hydrobromic acid.

7. Process according to claims 1-6, characterized in that the reaction is performed in methylene chloride.

8. Process according to claims 1-7, characterized in that the reaction is carried out at a temperature between - 20 and 30°C, preferably between -10 and 15°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-2 045 755 (PFIZER) <br> * Page 10, example 17; page 16, preparation K * & NL - A - 8001285 (Cat. D) <br><br> --- | 1,4 | C 07 D 499/00 |
| A | EP-A-0 073 674 (PFIZER) <br> * Claims * <br><br> ----- | 1 | |

|  |  |
|---|---|
|  | TECHNICAL FIELDS SEARCHED (Int. Cl. ³) <br><br> C 07 D 499/00 |

The present search report has been drawn up for all claims

| Place of search <br> THE HAGUE | Date of completion of the search <br> 14-06-1984 | Examiner <br> CHOULY J. |
|---|---|---|